# EUROPEAN PATENT SPECIFICATION

(11) **EP 4 142 943 B1**
(45) Date of publication and mention of the grant of the patent: **03.09.2025**
(21) Application number: 21796284.4
(22) Date of filing: 26.04.2021
(51) Int. Cl.: B01L 3/00, G05B 19/042, H04B 1/707, H04B 1/7097, H04B 17/24, H04L 27/26, G01N 33/493, A61B 10/00

(54) **INTERFERING FLAG TO REDUCE FALSES DIAGNOSIS DUE TO INTERFERING CONDITIONS IN MICROSCOPIC MORPHOLOGY BASED SEDIMENT URINALYSIS**
INTERFERIERENDE FLAGGE ZUR REDUZIERUNG VON FALSCHDIAGNOSEN AUFGRUND VON STÖRENDEN BEDINGUNGEN IN DER AUF MIKROSKOPISCHER MORPHOLOGIE BASIERENDEN SEDIMENT-URILYSE
INDICATEUR D'INTERFÉRENCE POUR RÉDUIRE LES ERREURS DE DIAGNOSTIC LIÉES À DES CONDITIONS D'INTERFÉRENCE DANS UNE URINALYSE SÉDIMENTAIRE BASÉE SUR LA MORPHOLOGIE MICROSCOPIQUE

(30) Priority: 28.04.2020 US 202063016514 P
(43) Date of publication of application: 08.03.2023
(73) Proprietor: Siemens Healthcare Diagnostics, Inc., Tarrytown, NY 10591 (US)
(72) Inventor: DAS, Kausik, Stoughton, Massachusetts 02072 (US)
(74) Representative: Schweitzer, Klaus
(86) International application number: PCT/US2021/029108
(87) International publication number: WO 2021/222076

(56) References cited:
- EP-A1- 2 772 745
- WO-A1-2009/094761
- WO-A1-2019/046115
- US-A1- 2003 022 390
- US-A1- 2011 118 141
- US-A1- 2012 115 174
- US-A1- 2015 359 522
- US-A1- 2018 348 200
- US-A1- 2018 348 200
- US-B1- 6 466 320
- US-B1- 7 072 618

## Description

### CROSS REFERENCE TO RELATED APPLICATIONS

This application claims benefit under 35 USC § 119(e) of US Provisional Application No. 63/016,514, filed April 28, 2020.

### STATEMENT REGARDING FEDERALLY FUNDED RESEARCH OR DEVELOPMENT

Not Applicable.

### BACKGROUND

Urinalysis can provide valuable information to aid in the diagnosis and treatment of patients suspected of various metabolic, renal and urinary tract disorders. Urine samples of such suspected patients may contain various analytes that are indicative of a pathological condition or stage of disease progression. For example, urine samples from patients with kidney stones or urinary tract injury contain red blood cells; urine samples from patients with various inflammatory conditions or urinary tract infections contain white blood cells and/or bacteria, Accurate and timely results of urinalysis is critical for patient care.

Urinalysis, in general, is the physical, chemical, microscopic and microbiological examinations of urine. Urinalysis involves a number of tests to detect and measure various analytes that pass through urine. Physical examination of urine samples provides information regarding its appearance, such as color, odor, and turbidity. Chemical examination of urine provides information regarding acidity (pH), specific gravity, and presence of proteins, sugars, ketones, bilirubin, nitrite, white blood cell products (leukocyte esterase), and blood. Microscopic examination of urine provides information regarding sediments present in urine, such as, for example, red blood cells, white blood cells, bacteria, epithelial cells, pathological cast, hyaline cast, crystal, yeast, mucus, and sperm. Microbiological examination of urine provides information regarding various pathogenic organisms.

Microscopic examination includes microscopy-based sediment urinalysis, which is a common detection technology utilized by clinical laboratories. Microscopy-based urine sediment analyzers evaluate urine samples for the presence of analytes based on an analyte's unique morphological characteristics including size, shape, and textural appearance. Microscopy-based detection technologies depend entirely on the morphological aspects of the formed elements present in a urine sample-no chemical reactions are involved in microscopy-based detection of urine sediment particles. Urine sediment elements may be detected manually using microscopy, or detected automatically using various automated sediment urine analyzers that utilize microscopy-based image technology.

However, appropriate measures are required to ensure that the sediment analyzers are properly detecting analytes based upon their morphologies. Circumstances such as interference may lead to false-positive or false-negative urinalysis results. Interference in detection of a particular urine analyte occurs due to the presence of another analyte with similar morphology. Certain analytes are, by nature, morphologically similar to other analytes. For example, ovoid shaped calcium oxalate-monohydrate and single cell yeasts can morphologically resemble red blood cells in urine samples. Consequently, the presence of yeast, for example, could lead to a false increase in red blood cells detected or vice versa. Further, certain analytes may undergo morphological changes under certain conditions which render them morphologically similar to other analytes. For example, crenation may occur in red blood cells in low pH urine mediums, or under high osmotic pressure, leaving the red blood cells almost indistinguishable from white blood cells.

Currently, interference issues are detected through visual inspection by a urinalysis expert, which is then confirmed or rejected using supporting or secondary analyses, such as a chemical treatment or staining. For example, chemicals or stains may be added to urine samples to distinguish interferents. Without visual inspection or without proper understanding of morphologies, interference issues may remain undetected. After initial detection, a manual microscopy user must rely on supporting methods to confirm or reject a suspected interference, such as by use of phase contrast microscopy or by staining the sample. However, this process is only utilized in manual microscopy. A user of an automated sediment analyzer, where results are sent directly for reporting, would have no knowledge of the interfering conditions without also having results from chemistry urinalysis. An automated sediment analyzer user would also not be able to confirm or reject the results without taking further steps, such as by staining the sample, applying a chemical treatment, or by retrospective review of the microscopic images of the sample. Therefore, improved quality control methods and/or systems for interference detection that do not require manual inspection and/or additional or secondary analyses are needed. It is to such improved methods and/or systems for interference detection that the present disclosure is directed.

US 2011/118141 A1 discloses a disease specific panel - which typically is a reagent strip - having at least one primary test for different analytes that are relevant for either early detection of a primary disease or management of patients already diagnosed with said primary disease. The panel also includes at least one secondary test which is relevant for detection of a co-morbid condition or complications of the primary disease. US 2011/118141 A1 may also provide flags or warnings notifying the operator of possible interferences with one or more of the reagent test results.

US 2018/348200 A1 discloses a urine analyzer, which includes an analysis unit for detecting fat particles in a measurement sample. Depending on the presence or absence of fat particles - determined by a threshold - the analyzer does or does not by-pass certain analytical procedures.

US 2012/115174 A1 discloses a test strip, which includes at least two tests, one of which is a urinary trypsin inhibitor (uTi) test, which comprises a specifically selected uTi reagent, which does not interfere with reactions, i.e. reagents on other reagent pads or vice versa on the test strip.

EP 2 772 745 A1 discloses a urine analyzer, which is capable of operating in a urine measurement mode and a body fluid measurement mode, wherin in the urine measurement mode a first measurement specimen is prepared by mixing a first aliquot of a urine sample and a first reagent, and a second measurement specimen is prepared by mixing a second aliquot of the urine sample and a second reagent having a hemolytic effect, and the first and second measurement specimens are supplied to a detecting section, wherein the signals of the first measurement specimen determine red blood cells and the signals of the second measurement specimen determine bacteria, and wherein under the body fluid measurement mode a third measurement specimen is prepared by mixing a first aliquot of body fluid sample and a first reagent, and a fourth measurement specimen is prepared by mixing a second aliquot of body fluid sample and a second reagent, and the third and fourth measurement specimens are supplied to a detecting section, wherein the signals of the third measurement specimen determine red blood cells in the body fluid sample and the signals of the fourth measurement specimen determine white blood cells in the body fluid sample.

### DETAILED DESCRIPTION

Before explaining at least one embodiment of the inventive concept(s) in detail by way of exemplary drawings, experimentation, results, and laboratory procedures, it is to be understood that the inventive concept(s) is not limited in its application to the details of construction and the arrangement of the components set forth in the following description or illustrated in the drawings, experimentation and/or results. The inventive concept(s) is capable of other embodiments or of being practiced or carried out in various ways. As such, the language used herein is intended to be given the broadest possible scope and meaning; and the embodiments are meant to be exemplary-not exhaustive. Also, it is to be understood that the phraseology and terminology employed herein is for the purpose of description and should not be regarded as limiting. In the following detailed description of embodiments of the inventive concept, numerous specific details are set forth in order to provide a more thorough understanding of the inventive concept. It will be apparent to one of ordinary skill in the art, however, that the inventive concept within the disclosure may be practiced without these specific details. In other instances, well-known features have not been described in detail to avoid unnecessarily complicating the instant disclosure.

Unless otherwise defined herein, scientific and technical terms used in connection with the presently disclosed and/or claimed inventive concept(s) shall have the meanings that are commonly understood by those of ordinary skill in the art. Further, unless otherwise required by context, singular terms shall include pluralities and plural terms shall include the singular. The foregoing techniques and procedures are generally performed according to conventional methods well known in the art and as described in various general and more specific references that are cited and discussed throughout the present specification. The nomenclatures utilized in connection with, and the laboratory procedures and techniques of, analytical chemistry, synthetic organic chemistry, and medicinal and pharmaceutical chemistry described herein are those well-known and commonly used in the art. Further, unless expressly stated to the contrary, "or" refers to an inclusive or and not to an exclusive or. For example, a condition A or B is satisfied by anyone of the following: A is true (or present) and B is false (or not present), A is false (or not present) and B is true (or present), and both A and B are true (or present).

All patents, published patent applications, and non-patent publications mentioned in the specification are indicative of the level of skill of those skilled in the art to which this presently disclosed and/or claimed inventive concept(s) pertains.

All of the systems, and/or methods disclosed and/or claimed herein can be made and executed without undue experimentation in light of the present disclosure. While the methods of this presently disclosed and/or claimed inventive concept(s) have been described in terms of preferred embodiments, it will be apparent to those of skill in the art that variations may be applied to the compositions and/or methods and in the steps or in the sequence of steps of the method described herein.

As utilized in accordance with the present disclosure, the following terms, unless otherwise indicated, shall be understood to have the following meanings:

The use of the word "a" or "an" when used in conjunction with the term "comprising" in the claims and/or the specification may mean "one," but it is also consistent with the meaning of "one or more," "at least one," and "one or more than one." The singular forms "a," "an," and "the" include plural referents unless the context clearly indicates otherwise. Thus, for example, reference to "a compound" may refer to 1 or more, 2 or more, 3 or more, 4 or more, or greater numbers of compounds. The term "plurality" refers to "two or more."

The use of ordinal number terminology (i.e., "first," "second," "third," "fourth," etc.) is solely for the purpose of differentiating between two or more items and, unless explicitly stated otherwise, is not meant to imply any sequence or order or importance to one item over another or any order of addition, for example.

The use of the term "or" in the claims is used to mean "and/or" unless explicitly indicated to refer to alternatives only or the alternatives are mutually exclusive, although the disclosure supports a definition that refers to only alternatives and "and/or." For example, a condition "A or B" is satisfied by any of the following: A is true (or present) and B is false (or not present), A is false (or not present) and B is true (or present), and both A and B are true (or present).

Throughout this application, the term "about" is used to indicate that a value includes the inherent variation of error for the device, the method being employed to determine the value, or the variation that exists among the study subjects. For example, but not by way of limitation, when the term "about" is utilized, the designated value may vary by ± 20% or ± 10%, or ± 5%, or ± 1%, or ± 0.1% from the specified value, as such variations are appropriate to perform the disclosed methods and as understood by persons having ordinary skill in the art.

The use of the term "at least one" will be understood to include one as well as any quantity more than one, including but not limited to, 2, 3, 4, 5, 10, 15, 20, 30, 40, 50, 100, etc. The term "at least one" may extend up to 100 or 1000 or more, depending on the term to which it is attached; in addition, the quantities of 100/1000 are not to be considered limiting, as higher limits may also produce satisfactory results. In addition, the use of the term "at least one of X, Y, and Z" will be understood to include X alone, Y alone, and Z alone, as well as any combination of X, Y, and Z. The use of ordinal number terminology (i.e., "first", "second", "third", "fourth", etc.) is solely for the purpose of differentiating between two or more items and is not meant to imply any sequence or order or importance to one item over another or any order of addition, for example.

Further, use of the term "plurality" is meant to convey "more than one" unless expressly stated to the contrary.

As used in this specification and claim(s), the terms "comprising" (and any form of comprising, such as "comprise" and "comprises"), "having" (and any form of having, such as "have" and "has"), "including" (and any form of including, such as "includes" and "include"), or "containing" (and any form of containing, such as "contains" and "contain") are inclusive or open-ended and do not exclude additional, unrecited elements or method steps.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AAB, BBC, AAABCCCC, CBBAAA, CABABB, and so forth. The skilled artisan will understand that typically there is no limit on the number of items or terms in any combination, unless otherwise apparent from the context.

As used herein, the term "substantially" means that the subsequently described event or circumstance completely occurs or that the subsequently described event or circumstance occurs to a great extent or degree. For example, the term "substantially" means that the subsequently described event or circumstance occurs at least 90% of the time, or at least 95% of the time, or at least 98% of the time.

As used herein any reference to "one embodiment" or "an embodiment" means that a particular element, feature, structure, or characteristic described in connection with the embodiment is included in at least one embodiment. The appearance of the phrase "in one embodiment" in various places in the specification are not necessarily all referring to the same embodiment. Further, all references to one or more embodiments or examples are to be construed as non-limiting to the claims.

The term "patient" includes human and veterinary subjects. In certain embodiments, a patient is a mammal. In certain other embodiments, the patient is a human, "Mammal" for purposes of treatment refers to any animal classified as a mammal, including human, domestic and farm animals, nonhuman primates, and zoo, sports, or pet animals, such as dogs, horses, cats, cows, etc.

As used herein, the term "processor" may refer to one or more units for processing including (for example but not by way of limitation) an ASIC, microcontroller, FPGA, microprocessor, digital signal processor (DSP) capability, state machine, or other suitable component. A processor may be configured to execute a computer program, e.g. as contained in machine readable instructions stored on a non-transitory computer readable memory and/or programmable logic. The processor may have various arrangements corresponding to those discussed for the circuitry, e.g. on-board and/ or off board the apparatus as part of the system.

Interference in detection of urine sediment particles, using microscopy-based image technology, may occur when other particles having similar morphologies are present in the same test sample. The presently disclosed and/or claimed inventive concept(s) relate generally to a system and method for detecting the presence of interfering conditions in the results of urine sediment analysis. More specifically, the presently disclosed and/or claimed inventive concept(s) relates to an improved system configured to generate one or more interfering flags based on testing apparatus detection results and methods of use related thereto. Such improved system(s) and method(s) allow for accurate urinalysis reporting. Detection of interfering conditions in a urine sample may be accomplished by analysis of a patient's urine sample by the testing apparatus where the detection measurements of a particular analyte are then compared to the detection measurements of another morphologically similar analyte. When the detection results of a patient's urine sample indicate that presence of one or more morphologically similar analytes at a particular concentration, the user should be alerted that the analytical results obtained from the testing apparatus may reflect false-positives or false negatives indicative of an interfering condition in the urine sample. The detection results of the one or more morphologically similar analytes may be represented by a range of acceptable values and/or upper and/or lower limits. However, when the detection results of the urine sediment analyzer do not detect the presence of one or more morphologically similar analytes, it is unlikely that an interfering condition is present in the urine sample.

According to the present invention an interfering flag is generated to alert a user of a testing apparatus of a possible interfering condition in the urine sample which may result in false detection of particular analytes.

In a first embodiment of the invention a system according to independent claim 1 is provided.

The inventive concepts disclosed herein may employ any morphology-based method or system as the testing apparatus to identify, classify, and count the formed elements in a urine sample using morphological methods. A variety of methods and analyzer systems for detecting analytes are well known in the art and include, but are not limited to, manual microscopy methods and automated analyzers utilizing microscopy-based detection technology. In certain non-limiting embodiments, the testing apparatus is an automated urinalysis analyzer. Non-limiting examples of automated urine sediment analyzers that can be utilized in accordance with the present disclosure include Atellica^{®} 1500 Automated Urinalysis System from Siemens Healthcare GmbH (Germany), UriSed2 from 77 Elektronika (Hungary), UriSed3 Pro from 77 Elektronika (Hungary), iQ200 Series Urinalysis Microscopy Analyzers from Beckman Coulter, Inc. (United States of America), sediMAX conTRUST Pro from A. Menarini Diagnostics (Italy), cobas^{®} 6500 urine analyzer series from Roche Diagnostics (Switzerland), and cobas^{®} u 701 microscopy analyzer from Roche Diagnostics (Switzerland). In certain non-limiting embodiments, the automated urinalysis analyzer is equipped with both chemistry and sediment analyzers. Automated analyzers identify, classify, and count a variety of analytes including, but not limited to, red blood cells, white blood cells (including white blood cell clumps), bacteria (including, but not limited to, rod and coccus bacteria), squamous epithelial cells, non-squamous epithelial cells, pathological casts, hyaline casts, crystals, yeast, mucus, and sperm. Automated analyzers may also detect other characteristics of urine samples including, but not limited to, clarity, color, pH, specific gravity, bilirubin, glucose, ketone, leukocyte esterase, nitrite, protein, urobilinogen, albumin, creatine, albumin-to-creatine ratio, and protein-to-creatine ratio. In certain non-limiting embodiments, the automated urinalysis analyzer is an image-based, automated urinalysis analyzer.

A urine-sample may be analyzed by an image-based, automated urinalysis analyzer. Detection results of the urine sample are obtained and the presence and concentration of analytes is determined. A user-configurable interference validation condition is selected based on the presence of one or more morphologically similar analytes. The presence and concentrations of both morphologically similar analytes are compared to the user-configurable interference validation condition. If the concentrations of both morphologically similar analytes meet the user-configurable interference validation condition, then an interfering flag is generated to alert the user of the automated urinalysis analyzer that an interfering condition is present in the urine sample. In certain non-limiting embodiments, the interfering flag may operate to block reporting of the detection results of the automated analyzer. Following the generation of an interfering flag, a user of the automated urinalysis analyzer may utilize manual microscopy methods to confirm or reject the suspected interference, such as, for example, adding glacial acetic acid to the urine sample to lyse the red blood cells to aid in differentiation.

A urine-sample may be analyzed by an automated urinalysis analyzer equipped with both chemistry and sediment analyzers. Sediment detection results of the urine sample are obtained and the presence of analytes is determined. Similarly, chemistry results of the urine sample are obtained and the presence or absence of elements and/or analytes is determined, for example the absence of blood. A user-configurable interference validation condition is selected based on the presence or absence of particular biochemically related elements and/or analytes, for example, red blood cells and blood. The presence, absence, and/or concentrations of both biochemically related elements and/or analytes are compared to the user-configurable interference validation condition. If the presence, absence, and/or concentrations of both biochemically related elements and/or analytes meet the user-configurable interference validation condition, then an interfering flag is generated, for example, a Yeast/RBC flag, to alert the user of the automated urinalysis analyzer that an interfering condition is present in the urine sample, such as, for example, the false detection of yeast. In certain non-limiting embodiments, the interfering flag may operate to block the reporting of the detection results of the automated analyzer. Following the generation of an interfering flag, a user of the automated urinalysis analyzer may utilize manual microscopy methods to confirm or reject the suspected interference.

The system may comprise a system processor and memory that collect, process, and store data from the testing apparatus. The system processor retrieves and executes instructions stored in the memory to control the operation of the system. In some non-limiting embodiments, morphological aspects of analytes that may be present in urine may be catalogued within the memory for retrieval and/or analysis. In some embodiments, interference validation conditions may be catalogued within the memory for retrieval and/or analysis. Systems and methods for detecting the presence of interfering conditions in the results from a urinalysis analyzer may also store and retrieve data from the memory, which may include the following morphological aspects of analytes that may be present in urine.

Red blood cells morphologically appear as pale reddish yellow, disk-shaped cells, with a diameter of in a range of from about 6 µm to about 8 µm and a dimple in the center. Red blood cells are detected and measured in a patient's urine sample to determine presence of any injury or inflammation along urinary tracts. For patients without any pathological conditions, the normal range for red blood cells is 0-3 red blood cells per high power field (RBC/HPF) or 3-12 RBC/µL (Brunzel, Nancy A. Fundamentals of Urine & Body Fluid Analysis. 4th ed., Elsevier, 2018). Red blood cell counts greater than 3 RBC/HPF or 12 RBC/ µL may be indicative of a pathological condition. Red blood cells, however, may undergo morphological changes under certain conditions, such as, for example (but not by way of limitation), when subjected to high osmotic pressure or when present in low pH urine medium.

Yeast are colorless ovoid cells of fungi, commonly of *Candida albicans* or rarely *Candida parapsilosis* or *Candida tropicalis* species. Yeast cells could be single or budding multiple cells. In general, these cells could vary in size in the range of from about 4 µm to about 10 µm. The presence of yeast in a patient's urine sample may indicate a medical condition, for example, urinary tract or bladder infection. Single cell yeast may appear morphologically similar to red blood cells or white blood cells.

Crystals may form in urine and may be indicative of various pathological conditions, including formation of kidney stone. Common crystals found in urine include calcium oxalate, uric acid, sodium urate, xanthine, cystine, and triple phosphate. Crystals may also vary in shape; for example (but not by way of limitation), crystals may be bipyramidal, ovoid, amorphous, or elongated. Crystals found in urine may also refer to undissolved particles or solid particles both crystalline and amorphous in nature. Certain crystals may appear morphologically similar to other analytes present in a patient's urine sample; for example (but not by way of limitation), ovoid shaped calcium oxalate-monohydrate crystals can resemble red blood cells in a patient's urine sample, and a cluster of amorphous crystals may resemble the coarse interior of granular cast.

White blood cells, morphologically, appear as spherical cells that have a diameter in a range of from about 10 µm to about 15 µm and a granular interior. For patients without any pathological conditions, the normal range for white blood cells is 0-8 WBC/HPF or 0 -10 WBC/µL (Brunzel, Nancy A. Fundamentals of Urine & Body Fluid Analysis. 4th ed., Elsevier, 2018). White blood cell counts greater than 8 WBC/HPF or 10 WBC/µL may be indicative of a pathological condition (Brunzel, Nancy A. Fundamentals of Urine & Body Fluid Analysis. 4th ed., Elsevier, 2018). White blood cells (WBCs), however, may undergo morphological changes under certain conditions, such as, for example (but not by way of limitation), due to aging. Aging white blood cells may resemble budding yeast in a patient's urine sample.

Casts are cylindrical structures are composed of uromodulin glycoprotein; casts are formed within urinary tubules and appear in a patient's urine sample. Casts are detected and measured in a patient's urine sample to determine a patient's kidney health. Hyaline casts are casts without any inclusion and appear translucent. Occasionally, however, hyaline casts may have solid particles (usually contaminants) stuck to them if the urine matrix contains amorphous particles. Pathological casts are casts that have various inclusions, such as (but not limited to) red blood cells, white blood cells, fat, etc., depending on a patient's particular pathological condition. Non-limiting examples of pathological casts include granular casts, cellular casts (red blood cells, white blood cells, epithelial cells), and waxy casts. The presence or absence of hyaline casts and/or pathological casts may be indicative of various kidney diseases. For example (but not by way of limitation), pathological casts with fat inclusions may indicate a nephrotic syndrome, or pathological casts with red blood cell inclusions may indicate microscopic bleeding from the kidneys. As such, it is clinically desirable to accurately detect the presence of hyaline and pathological casts to effectively diagnose and treat a patient's particular pathological condition. Hyaline casts with amorphous particle inclusions may appear morphologically similar to pathological casts. In this case, the interfering flag (pathological positive-hyaline negative) will not be sufficient if interfering flag is only designed to be triggered by Pathological positive and hyaline negative. False identification of pathological casts is usually found when solid particles are present in high amount (no particular threshold) and urine samples are mucoid or contain epithelial cells where solid particles can get stuck to give granular appearance of pathological cast interior.

Mucus is a viscous substance that coats certain parts of the body, including the urinary tract. While a small amount of mucus in urine is normal, an excess amount of mucus may indicate a urinary tract infection or other medical condition. Mucus strands may have indistinguishable appearance from hyaline casts in wet preparations of urine samples, because hyaline casts have a smooth texture and a refractive index very close to that of the surrounding fluids, such as mucus.

Bacteria may also be present in a patient's urine sample and may be indicative of an infection. Although a variety of bacteria may be present in the urine of a patient suffering from an infection, the most common strains include, for example (but not by way of limitation), *Escherichia coli (E. coll), Proteus, Klebsiella, Enterobacter, Staphylococcus,* and *Acinetobacter.*

The memory of the testing apparatus also stores instructions that, when executed by the system processor, cause the system processor to select a user-configurable interference validation condition and compare a first detection measurement to the selected user-configurable interference validation condition. The memory may also store instructions that, when executed by the system processor, cause the system processor to compare a second detection measurement to the selected user-configurable interference validation condition. In certain non-limiting embodiments, the user-configurable interference validation condition may comprise a first interference threshold level for a first analyte, a second interference threshold level for the second analyte, or both. The memory may include one or more of the following interference validation conditions.

A Yeast/WBC flag may alert the user of a testing apparatus that an interfering condition is present in the urine sample when the testing apparatus detects levels of yeast and levels of white blood cells that exceed a predetermined threshold level. The predetermined threshold level of yeast for a Yeast/WBC flag may be 1 yeast/HPF or 1 Yeast/µL. The predetermined threshold level of WBC for a Yeast/WBC flag may be 1 WBC/HPF or 1 WBC/µL. In certain non-limiting embodiments, the Yeast/WBC interfering flag may operate to block reporting of the detection results of the testing apparatus.

A Calcium Oxalate Crystal/RBC flag may alert the user of a testing apparatus that an interfering condition is present in the urine sample when the testing apparatus detects levels of calcium oxalate crystal and red blood cells that exceed a predetermined threshold level. The predetermined threshold level of calcium oxalate crystal for a Calcium Oxalate Crystal/RBC flag may be 1 Crystal/HPF or 1 crystal/µL. The predetermined threshold level of RBC for a Calcium Oxalate Crystal/RBC flag may be 1 RBC/HPF or 1 RBC/µL. In certain non-limiting embodiments, the Calcium Oxalate Crystal/RBC interfering flag may operate to block reporting of the detection results of the testing apparatus.

A Calcium Oxalate Crystal/RBC flag may alert the user of a testing apparatus equipped with both chemistry and sediment analyzers that an interfering condition is present in the urine sample when the chemistry analyzer of the testing apparatus fails to detect blood in a urine sample, but where the sediment analyzer detects red blood cells that exceed a predetermined threshold level. The predetermined threshold level of RBC for a Calcium Oxalate Crystal/RBC flag may be 1 RBC/HPF or 1 RBC/µL. In certain non-limiting embodiments, the Calcium Oxalate Crystal/RBC interfering flag may operate to block reporting of the detection results of the testing apparatus.

A Calcium Oxalate Crystal/RBC flag may alert the user of a testing apparatus equipped with both chemistry and sediment analyzers that an interfering condition is present in the urine sample when the chemistry analyzer of the testing apparatus fails to detect blood in a urine sample with a predetermined pH but where the sediment analyzer detects red blood cells that exceed a predetermined threshold level. The predetermined pH of the urine sample for a Calcium Oxalate Crystal/RBC flag may be less than about 5. The predetermined threshold level of RBC for a Calcium Oxalate Crystal/RBC flag may be 1 RBC/HPF or 1 RBC/µL. In certain non-limiting embodiments, the Calcium Oxalate Crystal/RBC interfering flag may operate to block reporting of the detection results of the testing apparatus.

An Amorphous/Pathological flag may alert the user of a testing apparatus that an interfering condition is present in the urine sample when the testing apparatus detects levels of pathological cast that exceed a predetermined threshold level, but does not detect hyaline cast. The predetermined threshold level of pathological cast for an Amorphous/Pathological flag may be 1 pathological cast/HPF or 1 pathological cast/µL. The predetermined maximum level of hyaline cast for an Amorphous/Pathological flag may be 1 hyaline cast/HPF or 1 hyaline cast/µL. In certain non-limiting embodiments, the Amorphous/Pathological interfering flag may operate to block reporting of the detection results of the testing apparatus.

A Crystal/Pathological flag may alert the user of a testing apparatus that an interfering condition is present in the urine sample when the testing apparatus detects levels of crystal and/or pathological cast that exceed a predetermined threshold level. The predetermined threshold level of crystal for a Crystal/Pathological flag may be 1 crystal/HPF or 1 crystal/µL The predetermined threshold level of pathological cast for a Crystal/Pathological flag may be 1 pathological cast/HPF or 1 pathological cast/µL. In certain non-limiting embodiments, the Crystal/Pathological interfering flag may operate to block reporting of the detection results of the testing apparatus. In certain non-limiting embodiments, the Crystal/Pathological interfering flag may more specifically be an Amorphous/Granular Cast flag that may alert the user of a testing apparatus that an interfering condition is present in the urine sample when the testing apparatus detects levels of amorphous crystal cluster(s) and/or granular cast that exceed a predetermined threshold level. The predetermined threshold level of amorphous crystal cluster(s) for an Amorphous/Granular Cast flag may be 1 crystal/HPF or 1 crystal/µL (Brunzel, Nancy A. Fundamentals of Urine & Body Fluid Analysis. 4th ed., Elsevier, 2018). The predetermined threshold level of granular cast for an Amorphous/Granular Cast flag may be 1 granular cast/HPF or 1 granular cast/µL. In certain non-limiting embodiments, the Amorphous/Granular Cast flag may operate to block reporting of the detection results of the testing apparatus.

A Mucus/Hyaline flag may alert the user of a testing apparatus that an interfering condition is present in the urine sample when the testing apparatus detects levels of mucus and/or hyaline cast that exceed a predetermined threshold level. The predetermined threshold level of mucus for a Mucus/Hyaline flag may be 1 mucus/HPF or 1 mucus/µL. The predetermined threshold level of hyaline cast for a Mucus/Hyaline flag may be 1 hyaline cast/HPF or 1 hyaline cast/µL. In certain non-limiting embodiments, the Mucus/Hyaline interfering flag may operate to block reporting of the detection results of the testing apparatus.

A Crystal/Bacteria flag may alert the user of a testing apparatus that an interfering condition is present in the urine sample when the testing apparatus detects levels of crystal and/or bacteria that exceed a predetermined threshold level. The predetermined threshold level of crystal for a Crystal/Bacteria flag may be 1 crystal/HPF or 1 crystal/µL. The predetermined threshold level of bacteria for a Crystal/Bacteria flag may be 1 bacteria/HPF or 1 bacteria/µL. In certain non-limiting embodiments, the Crystal/Bacteria interfering flag may operate to block reporting of the detection results of the testing apparatus.

An Amorphous/Bacteria flag may alert the user of a testing apparatus equipped with both chemistry and sediment analyzers that an interfering condition is present in the urine sample when the sediment analyzer detects bacteria that exceeds a predetermined threshold level but does not detect white blood cells above a predetermined maximum level, and where the chemistry analyzer does not detect nitrite above a predetermined maximum level. The predetermined threshold level of bacteria for an Amorphous/Bacteria flag may be 1 bacteria/HPF or 1 bacteria/µL. The predetermined maximum level of WBC for an Amorphous/Bacteria flag may be 8 WBC/HPF or 10 WBC/µL (Brunzel, Nancy A. Fundamentals of Urine & Body Fluid Analysis. 4th ed., Elsevier, 2018). The predetermined maximum level of nitrite for an Amorphous/Bacteria flag may be the negative/positive threshold for that analyzer. In certain non-limiting embodiments, the Amorphous/Bacteria interfering flag may operate to block reporting of the detection results of the testing apparatus.

A Crenated RBC/WBC flag may alert the user of a testing apparatus equipped with both chemistry and sediment analyzers that an interfering condition is present in the urine sample when the chemistry analyzer of the testing apparatus detects blood in a urine sample with a predetermined specific gravity and where the sediment analyzer detects white blood cells that exceed a predetermined threshold level (Chu-Su et al. 2017, "Enhancing the Detection of Dysmorphic Red Blood Cells and Renal Tubular Epithelial Cells with a Modified Urinalysis Protocol," Scientific Reports, 7:40521). The predetermined specific gravity threshold level of the urine sample for a Crenated RBC/WBC flag, may be 1.010-1.040, or 1.010-1.025, or 1.010-1.022, or 1.022-1.040, or 1.022-1.025, or 1.025-1.040. In certain non-limiting embodiments, the predetermined specific gravity threshold level is 1.022-1.025. (See Turgeon, Mary Louise. Linné & Ringsrud's Clinical Laboratory Science - The Basic and Routine Techniques. 6th ed. Elsevier, 2012; *see also* Brunzel, Nancy A. Fundamentals of Urine & Body Fluid Analysis. 4th ed., Elsevier, 2018). The predetermined threshold level of RBC and WBC for a Crenated RBC/WBC flag may be 1 RBC/HPF or 1 RBC/µL and 1 WBC/HPF or 1 WBC/µL. In certain non-limiting embodiments, the Crenated RBC/WBC interfering flag may operate to block reporting of the detection results of the testing apparatus.

A Yeast/RBC flag may alert the user of a testing apparatus that an interfering condition is present in the urine sample when the testing apparatus detects levels of yeast and/or levels of white blood cells that exceed a predetermined threshold level. The predetermined threshold level of yeast for a Yeast/RBC flag may be 1 yeast/HPF or 1 yeast/µL The predetermined threshold level of RBC for a Yeast/RBC flag may be 1 RBC/HPF or 1 RBC/µL. In certain non-limiting embodiments, the Yeast/RBC interfering flag may operate to block reporting of the detection results of the testing apparatus.

An Aging WBC/Budding Yeast flag may alert the users of a testing apparatus that an interfering condition is present in the urine sample when the testing apparatus detects levels of yeast and/or levels of white blood cells that exceed a predetermined threshold level. The predetermined threshold level of WBC for an Aging WBC/Budding Yeast flag may be 1 WBC/HPF or 1 WBC/µL. The predetermined threshold level of yeast for an Aging WBC/Budding Yeast flag may be 1 yeast/HPF or 1 yeast/µL. In certain non-limiting embodiments, the Aging WBC/Budding Yeast interfering flag may operate to block reporting of the detection results of the testing apparatus.

The memory may also store information regarding the patient population. For example, patient biological information may be stored. In some embodiments, such information may be stored with measurements of data associated therewith, in addition to other metadata associated with the information and/or data (e.g., date, algorithms used). The memory may store instructions that, when executed by the system processor, cause the system processor to select patient data to be used to select the interference validation condition. For example, presence of yeast is more prevalent in female patients than in male patients. Therefore, the system may use gender specific limit of detection for that system to detect yeast and to trigger yeast related interference flags. (See Turgeon, Mary Louise. Linné & Ringsrud's Clinical Laboratory Science - The Basic and Routine Techniques. 6th ed. Elsevier, 2012).

The memory may also store information regarding the limit detections of various testing apparatuses. The memory may store instructions that, when executed by the system processor, cause the system processor to select limit detections of a specific testing apparatus to be used to select the interference validation condition. For example, the testing apparatus may detect a particular analyte if the analyte is present in the urine sample at a level above the limit detection selected for the testing apparatus and triggers the appropriate interference flag. On the contrary, the testing apparatus may detect a particular analyte below the limit of detection of a particular analyte and may not trigger the flag because the analyte may not be present in the sample.

The memory may also store information regarding the predetermined threshold levels of particular analytes. The memory may store instruction that, when executed by the system processor, cause the system processor to select predetermined threshold levels of particular analytes to be used to select the interference validation condition.

## Claims

1. A system for detecting the presence of interfering conditions in the results from a testing apparatus that measures particles included in a urine sample, comprising:
a testing apparatus that measures particles included in a urine sample, **characterized in that**
interference in detection of a particular urine analyte may occur due to the presence of another analyte with similar morphology; and
a system processor communicatively coupled with the testing apparatus, the system processor coupled to a memory storing instructions that, when executed by the system processor, cause the system processor to:
- receive detection results of a urine sample from the testing apparatus, the results including (i) a first detection measurement of a first analyte present in the urine sample and (ii) a second detection measurement of a second analyte present in the urine sample, wherein the second analyte is a morphologically similar analyte to the first analyte;
- select a user-configurable interference validation condition, comprising a condition based on the presence of a plurality of morphologically similar analytes;
- compare the first detection measurement to the selected user-configurable interference validation condition;
- compare the second detection measurement to the selected user-configurable interference validation condition;
- generate one or more interfering flags if each of the first detection measurement and the second detection measurement meet the selected user-configurable interference validation condition;
- store the generated interfering flag in the memory; and
- display the interfering flag.

2. The system of claim 1, wherein the testing apparatus is an automated urinalysis analyzer.

3. The system of claim 1, wherein the testing apparatus is equipped with both chemistry and sediment analyzers, preferably wherein the detection results comprise chemistry urinalysis results, sediment urinalysis results, or a combination thereof.

4. The system of claim 1, wherein the user-configurable interference validation condition comprises a first interference threshold level for the first analyte, wherein preferably
- the first interference threshold level for the first analyte is adjusted based on the gender of the patient from which the urine sample was collected, or
- the first interference threshold level for the first analyte is adjusted based on the testing apparatus, or
- the first detection measurement meets the selected user-configurable interference condition if the first detection measurement exceeds the first interference threshold level.

5. The system of claim 1, wherein the user-configurable interference validation condition comprises a second interference threshold level for the second analyte, wherein
- preferably the second interference threshold level for the second analyte is adjusted based on the gender of the patient from which the urine sample was collected, or
- preferably the second interference threshold level for the second analyte is adjusted based on the testing apparatus, or
- preferably the second detection measurement meets the selected user-configurable interference condition if the second detection measurement exceeds the second interference threshold level.

6. The system of claim 1, wherein the user-configurable interference validation condition comprises a first interference threshold level for the first analyte and a second interference threshold level for the second analyte, preferably wherein each of the first detection measurement and the second detection measurement meet the selected user-configurable interference validation condition if the first detection measurement exceeds the first interference threshold level and the second detection measurement exceeds the second interference threshold level.

7. The system of claim 1, wherein the interfering flag is selected from an Amorphous/Pathological Flag, Mucus/Hyaline Flag, Yeast/RBC Flag, Amorphous/Bacteria Flag, Calcium Oxalate Crystal/RBC Flag, Crenated RBC/WBC Flag, Amorphous/Granular Cast Flag, Aging WBC/Budding Yeast Flag, and combinations thereof.

8. The system of claim 1, wherein the first analyte is selected from red blood cells, white blood cells, bacteria, squamous epithelial cells, nonsquamous epithelial cells, pathological cast, hyaline cast, crystal, yeast, mucus, and sperm.

9. The system of claim 1, wherein the second analyte is selected from red blood cells, white blood cells, bacteria, squamous epithelial cells, nonsquamous epithelial cells, pathological cast, hyaline cast, crystal, yeast, mucus, and sperm.

10. A method comprising:
receiving detection results of a urine sample from a system according to claims 1-9;
determining a first detection measurement of a first analyte present in the urine sample;
determining a second detection measurement of a second analyte present in the urine sample,
selecting a user-configurable interference validation condition, comprising a condition based on the presence of a plurality of morphologically similar analytes;
comparing the first detection measurement to the selected user-configurable interference validation condition;
comparing the second detection measurement to the selected user-configurable interference validation condition;
**characterized by**
generating one or more interfering flags if each of the first detection measurement and the second detection measurement meet the selected user-configurable interference validation condition;
storing the generated interfering flag in the memory; and
displaying the interfering flag.

## Patentansprüche

1. System zum Detektieren des Vorhandenseins von Interferenzbedingungen in den Ergebnissen einer Testvorrichtung, die in eine Urinprobe eingeschlossene Partikel misst, umfassend:
eine Testvorrichtung, die in einer Urinprobe eingeschlossene Partikel misst, **dadurch gekennzeichnet, dass**
Interferenz bei der Detektierung eines bestimmten Urinanalyten aufgrund des Vorhandenseins eines anderen Analyten mit ähnlicher Morphologie auftreten kann; und
einen Systemprozessor, der kommunikativ an die Testvorrichtung gekoppelt ist, wobei der Systemprozessor mit einem Speicher gekoppelt ist, der Anweisungen speichert, die bei Ausführung durch den Systemprozessor veranlassen, dass der Systemprozessor Folgendes ausführt:
- Empfangen von Detektierungsergebnissen einer Urinprobe von der Testvorrichtung, wobei die Ergebnisse einschließen: (i) eine erste Detektierungsmessung eines ersten Analyten, der in der Urinprobe vorhanden ist, und (ii) eine zweite Detektierungsmessung eines zweiten Analyten, der in der Urinprobe vorhanden ist, wobei der zweite Analyt ein morphologisch ähnlicher Analyt wie der erste Analyt ist;
- Auswählen einer benutzerkonfigurierbaren Interferenzvalidierungsbedingung, umfassend eine Bedingung basierend auf dem Vorhandensein einer Vielzahl von morphologisch ähnlichen Analyten;
- Vergleichen der ersten Detektierungsmessung mit der ausgewählten benutzerkonfigurierbaren Interferenzvalidierungsbedingung;
- Vergleichen der zweiten Detektierungsmessung mit der ausgewählten benutzerkonfigurierbaren Interferenzvalidierungsbedingung;
- Generieren von einem oder mehreren Interferenz-Flags, falls jede von der ersten Detektierungsmessung und der zweiten Detektierungsmessung die ausgewählte benutzerkonfigurierbare Interferenzvalidierungsbedingung erfüllt;
- Speichern des generierten Interferenz-Flags in dem Speicher; und
- Anzeigen des Interferenz-Flags.

2. System nach Anspruch 1, wobei die Testvorrichtung ein automatisierter Analysator für die Urinanalyse ist.

3. System nach Anspruch 1, wobei die Testvorrichtung sowohl mit chemischen als auch mit Sedimentanalysatoren ausgestattet ist, wobei die Detektierungsergebnisse vorzugsweise chemische Urinanalyseergebnisse, Sedimenturinanalyseergebnisse oder eine Kombination davon umfassen.

4. System nach Anspruch 1, wobei die benutzerkonfigurierbare Interferenzvalidierungsbedingung einen ersten Interferenzschwellenwert für den ersten Analyten umfasst, wobei vorzugsweise
- der erste Interferenzschwellenwert für den ersten Analyten basierend auf dem Geschlecht des Patienten angepasst wird, von dem die Urinprobe entnommen wurde, oder
- der erste Interferenzschwellenwert für den ersten Analyten basierend auf der Testvorrichtung angepasst wird, oder
- die erste Detektierungsmessung die ausgewählte benutzerkonfigurierbare Interferenzbedingung erfüllt, falls die erste Detektierungsmessung den ersten Interferenzschwellenwert überschreitet.

5. System nach Anspruch 1, wobei die benutzerkonfigurierbare Interferenzvalidierungsbedingung einen zweiten Interferenzschwellenwert für den zweiten Analyten umfasst, wobei
- der zweite Interferenzschwellenwert für den zweiten Analyten vorzugsweise basierend auf dem Geschlecht des Patienten angepasst wird, von dem die Urinprobe entnommen wurde, oder
- der zweite Interferenzschwellenwert für den zweiten Analyten vorzugsweise basierend auf der Testvorrichtung angepasst wird, oder
- die zweite Detektierungsmessung vorzugsweise die ausgewählte benutzerkonfigurierbare Interferenzbedingung erfüllt, falls die zweite Detektierungsmessung den zweiten Interferenzschwellenwert überschreitet.

6. System nach Anspruch 1, wobei die benutzerkonfigurierbare Interferenzvalidierungsbedingung einen ersten Interferenzschwellenwert für den ersten Analyten und einen zweiten Interferenzschwellenwert für den zweiten Analyten umfasst, wobei vorzugsweise jede von der ersten Detektierungsmessung und der zweiten Detektierungsmessung die ausgewählte benutzerkonfigurierbare Interferenzvalidierungsbedingung erfüllt, falls die erste Detektierungsmessung den ersten Interferenzschwellenwert überschreitet und die zweite Detektierungsmessung den zweiten Interferenzschwellenwert überschreitet.

7. System nach Anspruch 1, wobei das Interferenz-Flag ausgewählt ist aus einem amorphen/pathologischen Flag, Schleim/Hyalin-Flag, Hefe/RBC (Erythrozyten)-Flag, amorphen/Bakterien-Flag, Calciumoxalatkristall/RBC-Flag, gekerbten RBC/WBC (Leukozyten)-Flag, amorphen/granulären Zylinder-Flag, alternden WBC/Sproßhefe-Flag und Kombinationen davon.

8. System nach Anspruch 1, wobei der erste Analyt ausgewählt ist aus Erythrozyten, Leukozyten, Bakterien, Plattenepithelzellen, nicht-Plattenepithelzellen, pathologischen Zylindern, Hyalinzylindern, Kristall, Hefe, Schleim und Sperma.

9. System nach Anspruch 1, wobei der zweite Analyt ausgewählt ist aus Erythrozyten, Leukozyten, Bakterien, Plattenepithelzellen, nicht-Plattenepithelzellen, pathologischen Zylindern, Hyalinzylindern, Kristall, Hefe, Schleim und Sperma.

10. Verfahren, umfassend:
Empfangen von Detektierungergebnissen einer Urinprobe von einem System nach den Ansprüchen 1-9;
Bestimmen einer ersten Detektierungsmessung eines ersten Analyten, der in der Urinprobe vorhanden ist;
Bestimmen einer zweiten Detektierungsmessung eines zweiten Analyten, der in der Urinprobe vorhanden ist,
- Auswählen einer benutzerkonfigurierbaren Interferenzvalidierungsbedingung, umfassend eine Bedingung basierend auf dem Vorhandensein einer Vielzahl von morphologisch ähnlichen Analyten;
- Vergleichen der ersten Detektierungsmessung mit der ausgewählten benutzerkonfigurierbaren Interferenzvalidierungsbedingung;
- Vergleichen der zweiten Detektierungsmessung mit der ausgewählten benutzerkonfigurierbaren Interferenzvalidierungsbedingung;
**gekennzeichnet durch**
- Generieren von einem oder mehreren Interferenz-Flags, falls jede von der ersten Detektierungsmessung und der zweiten Detektierungsmessung die ausgewählte benutzerkonfigurierbare Interferenzvalidierungsbedingung erfüllt;
Speichern des generierten Interferenz-Flags in dem Speicher; und
Anzeigen des Interferenz-Flags.

## Revendications

1. Système de détection de la présence de conditions d'interférence dans les résultats d'un appareil de test qui mesure les particules incluses dans un échantillon d'urine, comprenant :
un appareil d'analyse qui mesure les particules contenues dans un échantillon d'urine, **caractérisé en ce que**
une interférence dans la détection d'un analyte urinaire particulier peut se produire en raison de la présence d'un autre analyte présentant une morphologie similaire ; et
un processeur système couplé de manière communicative à l'appareil de test, le processeur système couplé à une mémoire stockant des instructions qui, lorsqu'elles sont exécutées par le processeur système, amènent le processeur système à :
- recevoir les résultats de la détection d'un échantillon d'urine par l'appareil de test, les résultats comprenant (i) une première mesure de détection d'un premier analyte présent dans l'échantillon d'urine et (ii) une seconde mesure de détection d'un second analyte présent dans l'échantillon d'urine, dans lequel le second analyte est un analyte morphologiquement similaire au premier analyte ;
- sélectionner une condition de validation d'interférence configurable par l'utilisateur, comprenant une condition basée sur la présence d'une pluralité d'analytes morphologiquement similaires ;
- comparer la première mesure de détection à la condition de validation d'interférence configurable par l'utilisateur ;
- comparer la deuxième mesure de détection à la condition de validation d'interférence configurable par l'utilisateur ;
- générer un ou plusieurs drapeaux d'interférence si la première mesure de détection et la seconde mesure de détection satisfont toutes deux à la condition de validation d'interférence configurable par l'utilisateur ;
- stocker le drapeau d'interférence généré dans la mémoire ; et
- afficher le drapeau d'interférence.

2. Système selon la revendication 1, dans lequel l'appareil de test est un analyseur d'urine automatisé.

3. Système selon la revendication 1, dans lequel l'appareil de test est équipé d'analyseurs de chimie et de sédiments, de préférence dans lequel les résultats de détection comprennent des résultats d'analyse chimique de l'urine, des résultats d'analyse de sédiments de l'urine ou une combinaison de ceux-ci.

4. Système selon la revendication 1, dans lequel la condition de validation d'interférence configurable par l'utilisateur comprend un premier niveau de seuil d'interférence pour le premier analyte, dans lequel de préférence
- le premier seuil d'interférence pour le premier analyte est ajusté en fonction du sexe du patient duquel l'échantillon d'urine a été prélevé, ou
- le premier seuil d'interférence pour le premier analyte est ajusté en fonction de l'appareil d'essai, ou
- la première mesure de détection répond à la condition d'interférence configurable par l'utilisateur si la première mesure de détection dépasse le premier seuil d'interférence.

5. Système selon la revendication 1, dans lequel la condition de validation d'interférence configurable par l'utilisateur comprend un second niveau de seuil d'interférence pour le second analyte, dans lequel
- de préférence, le second seuil d'interférence pour le second analyte est ajusté en fonction du sexe du patient duquel l'échantillon d'urine a été prélevé, ou
- de préférence, le second seuil d'interférence pour le second analyte est ajusté en fonction de l'appareil d'essai, ou
- de préférence, la deuxième mesure de détection répond à la condition d'interférence configurable par l'utilisateur sélectionnée si la deuxième mesure de détection dépasse le deuxième niveau de seuil d'interférence.

6. Système selon la revendication 1, dans lequel la condition de validation d'interférence configurable par l'utilisateur comprend un premier niveau de seuil d'interférence pour le premier analyte et un second niveau de seuil d'interférence pour le second analyte, de préférence dans lequel chacune de la première mesure de détection et de la seconde mesure de détection satisfait à la condition de validation d'interférence configurable par l'utilisateur sélectionnée si la première mesure de détection dépasse le premier niveau de seuil d'interférence et si la seconde mesure de détection dépasse le second niveau de seuil d'interférence.

7. Système selon la revendication 1, dans lequel le drapeau d'interférence est choisi parmi un drapeau amorphe/pathologique, un drapeau de mucus/hyaline, un drapeau de levure/BCR, un drapeau amorphe/bactérie, un drapeau de cristaux d'oxalate de calcium/BCR, un drapeau de globules rouges/BCR crénelés, un drapeau de coulées amorphes/granulaires, un drapeau de globules rouges vieillissants/de levure bourgeonnante, et des combinaisons de ceux-ci.

8. Système selon la revendication 1, dans lequel le premier analyte est choisi parmi les globules rouges, les globules blancs, les bactéries, les cellules épithéliales squameuses, les cellules épithéliales non squameuses, les coulées pathologiques, les coulées hyalines, les cristaux, les levures, le mucus et les spermatozoïdes.

9. Système selon la revendication 1, dans lequel le second analyte est choisi parmi les globules rouges, les globules blancs, les bactéries, les cellules épithéliales squameuses, les cellules épithéliales non squameuses, les coulées pathologiques, les coulées hyalines, les cristaux, les levures, le mucus et les spermatozoïdes.

10. Procédé comprenant les étapes consistant à :
recevoir des résultats de détection d'un échantillon d'urine provenant d'un système selon les revendications 1 à 9 ;
déterminer une première mesure de détection d'un premier analyte présent dans l'échantillon d'urine ;
déterminer une deuxième mesure de détection d'un deuxième analyte présent dans l'échantillon d'urine,
sélectionner une condition de validation d'interférence configurable par l'utilisateur, comprenant une condition basée sur la présence d'une pluralité d'analytes morphologiquement similaires ;
comparer la première mesure de détection à la condition sélectionnée de validation d'interférence configurable par l'utilisateur ;
comparer la deuxième mesure de détection à la condition sélectionnée de validation d'interférence configurable par l'utilisateur ;
caractériser par le fait qu'il comprend les étapes consistant à :
générer un ou plusieurs drapeaux d'interférence si la première mesure de détection et la seconde mesure de détection satisfont toutes deux à la condition sélectionnée de validation d'interférence configurable par l'utilisateur ;
stocker le drapeau d'interférence généré dans la mémoire ; et
afficher le drapeau d'interférence.
